# EUROPEAN PATENT APPLICATION

(11) **EP 3 659 581 A1**
(43) Date of publication of application: **03.06.2020**
(21) Application number: 18839236.9
(22) Date of filing: 23.07.2018
(51) Int. Cl.: A61K 8/60, A61K 31/7064, A61P 17/00, A61P 17/02, A61P 37/08, A61P 43/00, A61Q 19/08

(54) **TOPICAL COMPOSITION**

(30) Priority: 24.07.2017 JP 2017142718
(71) Applicant: Otsuka Pharmaceutical Co., Ltd., Tokyo 101-8535 (JP)
(72) Inventor: KAWAMURA, Mitsuaki, Osaka-shi Osaka 540-0021 (JP); TANAKA, Masahiko, Osaka-shi Osaka 540-0021 (JP); IGARASHI, Sachiyo, Osaka-shi Osaka 540-0021 (JP)
(74) Representative: Hoffmann Eitle
(86) International application number: PCT/JP2018/027449
(87) International publication number: WO 2019/021988

(57) **Abstract**

The invention provides a composition for external use comprising a phosphate ester of uridine or a physiologically acceptable salt thereof as an active ingredient for moisturizing, anti-wrinkling, anti-sagging, wound healing, prevention of hair loss, hair growth, or prevention/treatment of atopic dermatitis.

## Description

### Technical Field

The present application relates to a composition for external use for moisturizing, anti-wrinkling, anti-sagging, wound healing, prevention of hair loss, hair growth, prevention/treatment of atopic dermatitis, promotion of cell growth, and promotion of hyaluronic acid production. Furthermore, the present application relates to a method for increasing skin moisture retention, a method for reducing wrinkle, a method for reducing skin-sag, a method for treating a wound, a method for preventing hair loss, a method for promoting hair growth, a method for preventing/treating atopic dermatitis, a method for promoting cell growth, and a method for promoting hyaluronic acid production.

### Background Art

The skin originally has a moisturizing function, the sebum barrier on the skin surface prevents the evaporation of moisture from the body. Natural moisturizing factors such as amino acids and inorganic salts in the skin retain the moisture inside the skin. However when the skin ages or continues to be dry, the sebum and the amounts of the natural moisturizing factors decrease, and the moisturizing function of the skin declines.

Hyaluronic acid, which is one of the natural moisturizing factors, is a linear macromolecular polysaccharide formed by alternately combining β-D-N-acetylglucosamine and β-D-glucuronic acid, and has a high water retention ability. The skin has a structure having layers of epidermis, dermis, and subcutaneous tissue in order from the outside. Hyaluronic acid is mainly produced in keratinocytes in the epidermis and in fibroblasts in the dermis, and fills the extracellular space of the epidermis and the meshwork of the dermis, and thereby is deeply involved in maintaining the functions of cells and maintaining skin homeostasis. In the skin, hyaluronic acid is present in the dermis mainly, and the amount of hyaluronic acid in the skin decreases due to physiological aging.

Hyaluronic acid is used as a typical moisturizing ingredient in many cosmetics and the like. In addition to the high water retention ability, while the whole picture has not yet been clarified, hyaluronic acid has been reported to have many functions such as promoting tissue regeneration, and has been used in many uses such as treatment of wrinkle and skin-sag (Non-Patent Document 1 and 2), wound healing (Non-Patent Document 3 and 4), and treatment of atopic dermatitis (Non-Patent Document 5).

It is known that a molecular weight of 500 or less is desirable for the percutaneous absorption. However the molecular weight of hyaluronic acid generally used in cosmetics and the like is one million or more, thus it is difficult to deliver the hyaluronic acid into the skin inside by applying on the skin. That is, an agent for external use containing hyaluronic acid can moisturize the skin surface but cannot enhance the water retention ability of the skin inside. Therefore, for example, although the agent for external use containing hyaluronic acid is useful for epidermal wrinkles, they cannot be expected to be effective for dermal wrinkles, and an invasive procedure such as a direct injection of hyaluronic acid into the skin has been conducted for dermal wrinkles (Non-Patent Document 1) .

Cosmetics that enhance the ability of the skin to produce hyaluronic acid have been developed, and Patent Document 1 and Patent Document 2 disclose that acetylglucosamine, glucuronic acid and the like are substances that promote hyaluronic acid production. However, these documents disclose only the effect on the horny layer, which is the outermost layer of the epidermis, and the effect on the dermis where a large of amount of hyaluronic acid is present has not been confirmed.

In order to develop an agent for external use according to the user's condition, it is strongly desired that variations of substances which promote hyaluronic acid production are increased, and particularly it is strongly desired the substances which are effective not only on the cells of the skin epidermis but also on the cells of the skin dermis.

The dermal papilla cell is said to a commander of the hair growth signal and has been a main research object of the hair care research. Research regarding hair growth / hair loss focusing on the hair dermal papilla cell has been accumulated, and it is known that promoting the growth of dermal papilla cells brings about a hair growth effect. Drugs based on the promoting effect on the dermal papilla cell growth have been developed (for example, Patent Documents 6 and 7), however there is still a great need for new hair growth promoters and hair loss inhibitors.

Uridine monophosphate is one of the nucleotides and is a phosphate ester of uridine. Patent Document 2, Patent Document 3, and Patent Document 4 disclose that uridine monophosphate is used as a potentiator for an active ingredient. However none of these documents disclose that uridine monophosphate independently serves as an active ingredient.

Patent Document 5 discloses that the hyaluronic acid production was promoted in keratocytes treated with uridine. This patent document relates to a composition for preventing/treating dry eye syndrome, and is not directed to the promotion of hyaluronic acid production in the skin.

### Prior Art Documents

### Patent Document

Patent Document 1: JP 2001-2551 A
Patent Document 2: JP 2014-88329 A
Patent Document 3: WO 2003/084485
Patent Document 4: WO 2005/034902
Patent Document 5: JP 2009-517380 A
Patent Document 6: WO 2016/079912
Patent Document 7: JP 2015-13849 A

### Non-Patent Document

Non-Patent Document 1: Miyaji, 2 other authors, Advanced Cosmetic Dermatology 2, "Shiwa/tarumi wo toru kanja no manzokudo wo takameru tiryo no subete" [Remove wrinkles/sagging, All about treatments to increase patient satisfaction], Nankodo, June 2006, pp. 53-59 (in Japanese).
Non-Patent Document 2: Nobile V, Buonocore D, Michelotti A, Marzatico F (2014) Anti-aging and filling efficacy of six types hyaluronic acid based dermo-a cosmetic treatment: double blind, randomized clinical trial of efficacy and safety. J Cosmet Dermatol 13: 277-287.
Non-Patent Document 3: WYJ Chen, G Abatangelo (1999) Functions of hyaluronan in wound repair. Wound Repair and Regeneration, Vol 7, No. 2: 79-89
Non-Patent Document 4: Neuman, M.G.; Nanau, R.M.; Oruna-Sanchez, L.; Coto, G. Hyaluronic acid and wound healing. J. Pharm. Pharm. Sci. 2015, 18, 53-60.
Non-Patent Document 5: Draelos ZD. A clinical evaluation of the comparable efficacy of hyaluronic acid-based foam and ceramide-containing emulsion cream in the treatment of mild-to-moderate atopic dermatitis. J Cosmet Dermatol. 2011;10:185-188

The disclosures of the prior art documents cited in this description are all incorporated herein by reference.

### Summary of Invention

### Technical Problem

An object of the present application is to provide a composition for external use which promotes hyaluronic acid production in skin epidermis and skin dermis, and is useful for moisturizing, anti-wrinkling, anti-sagging, wound healing, prevention of hair loss, hair growth, or prevention/treatment of atopic dermatitis. Another object of the present application is to provide a method for increasing skin moisture retention, a method for reducing wrinkle, a method for reducing skin-sag, a method for treating a wound, a method for preventing hair loss, a method for promoting hair growth, and a method for preventing/treating atopic dermatitis.

### Solution to Problem

As a result of studies to solve the above problems, the present inventors have surprisingly found that a phosphate ester of uridine independently has an excellent effect on cell growth promotion, and can independently promote hyaluronic acid production in skin epidermis and skin dermis, thereby reaching the present invention.

The present invention provides the following:
[1] A composition for external use, comprising a phosphate ester of uridine or a physiologically acceptable salt thereof as an active ingredient for moisturizing, anti-wrinkling, anti-sagging, wound healing, prevention of hair loss, hair growth, or prevention/treatment of atopic dermatitis.
[2] The composition according to [1], comprising a phosphate ester of uridine or a physiologically acceptable salt thereof as an independent active ingredient.
[3] The composition according to [1] or [2], comprising no effective amount of any other active ingredient for moisturizing, anti-wrinkling, anti-sagging, wound healing, prevention of hair loss, hair growth, or prevention/treatment of atopic dermatitis.
[4] The composition according to any one of [1] to [3], wherein a phosphate ester of uridine or a physiologically acceptable salt thereof is selected from uridine monophosphate, uridine diphosphate, uridine triphosphate, and a physiologically acceptable salt thereof.
[5] The composition according to any one of [1] to [4], wherein a phosphate ester of uridine or a physiologically acceptable salt thereof is selected from uridine monophosphate and a physiologically acceptable salt thereof.
[6] The composition according to any one of [1] to [5], wherein a phosphate ester of uridine or a physiologically acceptable salt thereof is selected from uridine 5'- monophosphate and a physiologically acceptable salt thereof.
[7] The composition according to any one of [1] to [6], wherein the composition is for use on skin.
[8] The composition according to any one of [1] to [7], wherein the composition is used for promoting cell growth or promoting hyaluronic acid production.
[9] The composition according to any one of [1] to [8], which is used for a cosmetic, a drug for external use, or a quasi-drug for external use.
[10] A composition for external use, comprising a phosphate ester of uridine or a physiologically acceptable salt thereof as an active ingredient for promoting cell growth or promoting hyaluronic acid production.
[11] The composition according to [10], comprising a phosphate ester of uridine or a physiologically acceptable salt thereof as an independent active ingredient.
[12] The composition according to [10] or [11], comprising no effective amount of any other active ingredient for promoting cell growth or promoting hyaluronic acid production.
[13] The composition according to any one of [10] to [12], wherein a phosphate ester of uridine or a physiologically acceptable salt thereof is selected from uridine monophosphate, uridine diphosphate, uridine triphosphate, and a physiologically acceptable salt thereof.
[14] The composition according to any one of [10] to [13], wherein a phosphate ester of uridine or a physiologically acceptable salt thereof is selected from uridine monophosphate and a physiologically acceptable salt thereof.
[15] The composition according to any one of [10] to [14], wherein a phosphate ester of uridine or a physiologically acceptable salt thereof is selected from uridine 5'- monophosphate and a physiologically acceptable salt thereof.
[16] The composition according to any one of [10] to [15], wherein the composition is for use on skin.
[17] The composition according to any one of [10] to [16], which is used for a use selected from moisturizing, anti-wrinkling, anti-sagging, wound healing, prevention of hair loss, hair growth, and prevention/treatment of atopic dermatitis.
[18] The composition according to [17], comprising no effective amount of any other active ingredient for moisturizing, anti-wrinkling, anti-sagging, wound healing, prevention of hair loss, hair growth, or prevention/treatment of atopic dermatitis.
[19] The composition according to any one of [10] to [18], which is used for a cosmetic, a medical drug for external use, or a quasi-medical drug for external use.
[20] A method used for the purpose selected from the following (1) to (9), comprising applying a phosphate ester of uridine or a physiologically acceptable salt thereof to skin:
   (1) increasing skin moisture retention
   (2) reducing wrinkle
   (3) reducing skin-sag
   (4) treating a wound
   (5) preventing hair loss
   (6) promoting hair growth
   (7) preventing/treating atopic dermatitis
   (8) promoting cell growth
   (9) promoting hyaluronic acid production.
[21] A use of a phosphate ester of uridine or a physiologically acceptable salt thereof in the manufacture of a composition for external use comprising a phosphate ester of uridine or a physiologically acceptable salt thereof as an active ingredient for moisturizing, anti-wrinkling, anti-sagging, wound healing, prevention of hair loss, hair growth, prevention/treatment of atopic dermatitis, promotion of cell growth, or promotion of hyaluronic acid production.
[22] A phosphate ester of uridine or a physiologically acceptable salt thereof for use in moisturizing, anti-wrinkling, anti-sagging, wound healing, prevention of hair loss, hair growth, prevention/treatment of atopic dermatitis, promotion of cell growth, or promotion of hyaluronic acid production.

### Effect of The Invention

The composition of the present application can promote cell growth. The composition of the present application can promote hyaluronic acid production in skin epidermal cell and skin dermal cells, thereby improving skin moisture retention and skin flexibility, and maintaining a healthy and resilient skin. The composition of the present application can prevent hair loss or promote hair growth.

### Brief Description of Drawing

FIG. 1 depicts a graph showing the amount of hyaluronic acid produced when human epidermal keratinocytes were cultured in media containing various concentrations of UMP2Na or uridine in Test Example 1.
FIG. 2 depicts a graph showing the amount of hyaluronic acid produced when human dermal fibroblasts were cultured in media containing various concentrations of UMP2Na or uridine in Test Example 1.
FIG. 3 depicts a graph showing the number of cells when human dermal fibroblasts are cultured in media containing various concentrations of UMP2Na or uridine in Test Example 2.
FIG. 4 depicts a graph showing the number of cells when human dermal papilla cells are cultured in media containing various concentrations of UMP2Na in Test Example 3.
FIG. 5 depicts a graph showing the amount of hyaluronic acid produced when human dermal fibroblasts were cultured in media containing various concentrations of AMP2Na in Test Example 4.

### Description of Embodiment

The composition of the present invention comprises a phosphate ester of uridine or a physiologically acceptable salt thereof as an active ingredient for moisturizing, anti-wrinkling, anti-sagging, wound healing, prevention of hair loss, hair growth, prevention/treatment of atopic dermatitis, promotion of cell growth, or promotion of hyaluronic acid production.

In the present invention, an active ingredient means a substance that is independently capable of exerting a desired physiological action of the skin and is contained in a composition in the expectation that the composition containing the active ingredient exerts the desired physiological action. Examples of the desired physiological action include moisturizing, anti-wrinkling, anti-sagging, wound healing, prevention of hair loss, promotion of hair growth, prevention/treatment of atopic dermatitis, promotion of cell growth (for example, hyaluronic acid-producing cell (for example, keratinocyte, fibroblast), dermal papilla cell), and promotion of hyaluronic acid production. In the present invention, the active ingredient can independently exert an desired physiological action, and when used in combination with other ingredient(s), the desired physiological action may be enhanced additively or synergistically.

In the present invention, the wording "as an independent active ingredient" means that even without interaction with other active ingredient (s), a phosphate ester of uridine or a physiologically acceptable salt thereof may exert moisturizing effect, anti-wrinkling effect, anti-sagging effect, wound healing effect, hair loss prevention effect, hair growth promotion effect, atopic dermatitis prevention/treatment effect, promotion effect of cell growth (for example, hyaluronic acid-producing cell (for example, keratinocyte, fibroblast), dermal papilla cell), or promotion effect of hyaluronic acid production. The wording "as an independent active ingredient" does not preclude that the composition of the present invention comprises other active ingredient(s).

As used herein, "effective amount" means an amount that experts an effect of moisturizing, anti-wrinkling, anti-sagging, wound healing, prevention of hair loss, hair growth, prevention/treatment of atopic dermatitis, promotion of cell growth, or promotion of hyaluronic acid production.

As used herein, "other active ingredient(s) for moisturizing, anti-wrinkling, anti-sagging, wound healing, prevention of hair loss, hair growth, or prevention/treatment of atopic dermatitis" means ingredient(s) other than a phosphate ester of uridine or a physiologically acceptable salt thereof, known as an active ingredient for moisturizing, anti-wrinkling, anti-sagging, wound healing, prevention of hair loss, hair growth, or prevention/treatment of atopic dermatitis.

As used herein, "other active ingredient(s) for promoting cell growth or promoting hyaluronic acid production" means an ingredient other than a phosphate ester of uridine or a physiologically acceptable salt thereof, known as an active ingredient for promoting cell growth or promoting hyaluronic acid production.

As used herein, examples of a phosphate ester of uridine include uridine monophosphate (uridine 5'- monophosphate, uridine 3'-monophosphate, uridine 2'- monophosphate), uridine diphosphate, uridine triphosphate, uridine cyclic phosphate. Preferable examples of a phosphate ester of uridine include uridine monophosphate, more preferably uridine 5'-monophosphate.

Examples of a physiologically acceptable salt of a phosphate ester of uridine include alkali metal salts such as sodium salts, potassium salts and the like; alkaline earth metal such as calcium salts, magnesium salts, barium salts and the like; basic amino acid salts such as arginine, lysine and the like; ammonium salts such as ammonium salts, tricyclohexylammonium salts and the like; alkanolamine salts such as monoethanolamine salts, diethanolamine salts, triethanolamine salts, monoisopropanolamine salts, diisopropanolamine salts, triisopropanolamine salts and the like. Preferable salts includes alkali metal salts such as sodium salts. Examples of the alkali metal salts include uridine monophosphate monosodium salt, and uridine monophosphate disodium salt (hereinafter referred to as UMP2Na).

In the present invention, a phosphate ester of uridine or a physiologically acceptable salt thereof may be a single type of a phosphate ester of uridine or a salt thereof, or may be a mixture of a plurality of types of phosphate ester of uridine or a salt thereof.

The amount of a phosphate ester of uridine or a physiologically acceptable salt thereof contained in the composition of the present invention may vary depending on the type of a phosphate ester of uridine or a physiologically acceptable salt thereof, the use or form of the composition etc., but may be optionally selected from, for example, a range of usually 0.0001 to 20 % by weight, preferably 0.0001 to 10 % by weight, based on the total weight of the composition. Preferably from 0.001 % by weight to 10 % by weight, more preferably from 0.01 % by weight to 10 % by weight, more preferably from 0.01 % by weight to 5 % by weight, particularly preferably from 0.1 % by weight to 10 % by weight, particularly preferably from 0.5 % by weight to 5 % by weight, also preferably from 0.05 % by weight to 3 % by weight, and even more preferably from 0.1 % by weight to 1 % by weight are exemplified.

Further examples of the upper limit of the amount of a phosphate ester of uridine or a physiologically acceptable salt thereof contained in the composition of the present application include, relative to the total weight of the composition, preferably 10 % by weight, 7 % by weight, 5 % by weight, 3 % by weight, and 2 % by weight, and particularly preferably 1 % by weight.

Further examples of the lower limit of the amount of a phosphate ester of uridine or a physiologically acceptable salt thereof contained in the composition of the present application include, relative to the total weight of the composition, preferably 0.01 % by weight, 0.05 % by weight, 0.1 % by weight, 0.5 % by weight, and 0.7 % by weight, and particularly preferably 1 % by weight.

The compositions of the present invention may be prepared in a variety of forms in combination with a pharmaceutically or cosmetically acceptable bases or carriers, in addition to a phosphate ester of uridine or a physiologically acceptable salt thereof. As a pharmaceutically or cosmetically acceptable base or carrier, conventionally known ones may be used. The composition of the present invention may comprise, if required, a wide variety of known ingredients used for externally-applied compositions suitable for the skin and/or mucosa, such as cosmetics and externally-applied medical/quasi-medical drugs. Examples of such ingredients include surfactants, colorants (dyes, pigments)), flavors, antiseptics, bactericides (antibacterials), thickeners, antioxidants, sequestering agents, cooling agents, deodorizers, humectants, UV absorbers, UV dispersants, vitamins, plant extracts, astringents, anti-inflammatory agents (antiphlogistic agents), whiteners, cell activators, vasodilators, blood circulation accelerators, skin function accelerators, and the like.

Among the above ingredients, specific examples of the surfactant include anionic surfactants such as higher fatty acid soaps, alkyl sulfates, polyoxyethylene alkyl ether sulfates, alkyl ether phosphates, *N*-acylamino acid salts, acyl *N*-methyl taurine salts, and the like; cationic surfactants such as alkyltrimethylammonium chlorides, dialkyldimethylammonium chlorides and the like; amphoteric surfactants such as alkyldimethylaminoacetate betaines, alkylamidedimethylaminoacetate betaines, 2-alkyl-*N*-carboxy-N-hydroxyimidazolinium betaines and the like; nonionic surfactants such as polyoxyethylene bases, polyhydric alcohol ester bases, ethylene oxide/propylene oxide block copolymers and the like. Any high molecular weight surfactants or natural surfactants can also be used without limitation.

Examples of antiseptic include ethyl p-hydroxybenzoate, salicylic acid, sorbic acid and the like. Specific examples of thickener include xanthane gum, carboxymethyl cellulose sodium, carboxyvinyl polymers and the like. Specific examples of sequestering agent include sodium salts of ethylenediamine tetra acetic acid, phosphoric acid, citric acid and the like.

The composition of the present invention may be used as an externally-applied preparation to be spread or sprayed to the skin. More specifically, the composition of the present invention may be widely used as a cosmetic or an externally-applied medical drug, an externally-applied quasi-medical drug (dermatological preparation). Preferable among the above are cosmetics given that cosmetics can be used on a daily basis to bring about promotion of hyaluronic acid production in the skin on a daily basis. Examples of such externally-applied preparation include a wide variety of hair care products such as hair restorers and hair growth preparations, as well as, shampoos, rinses, and hair lotions (including tonics and liquids) that have hair restoration and/or hair growth effect(s).

When the composition of the present invention is used for hair growth or preventing hair loss, the composition of the present invention is preferably formulated into a hair cosmetic exemplified above. The composition of the present invention may also be used as an agent for promoting growth of /preventing loss of hairs such as eyebrows, eyelashes and the like which are desired to increase/elongate, in addition to head hairs. The wording "hair growth" means that the existing hair, including downy hair, grows healthy, and includes that a sprout of new hair is promoted. The wording "prevention of hair loss" means preventing loss of the existing hairs.

When the composition of the present invention is used for hair growth or prevention of hair loss, the composition of the present invention may be used for use associated with these (for example , prevention or treatment of thinning hair, promotion of hair emergence, hair growth promotion, treatment of post-illness/postpartum hair loss, hair restoration and the like).

The composition of the present invention may be any form without limitation insofar as it is applicable to the skin or mucosa. Examples of the form include pastes, mousses, gels, liquids, emulsions, suspensions, creams, ointments, solids, sheets, aerosols, sprays, and liniments. Examples of cosmetic include lotions; emulsions such as emollient emulsions, milky lotions, nourishing emulsions, cleansing emulsions and the like; creams such as emollient creams, massage creams, cleansing creams, makeup creams; lip creams and like. Examples of hair care product such as hair nourishing agents and hair restoring agents include hair tonics, hair creams, hair lotions, aerosols (air sprays), mousses, shampoos, rinses, liquids and the like.

The composition of the present invention may be directly applied to or sprayed onto the skin or mucosa as a cosmetic or an externally-applied medical/quasi-medical drug. The composition can be applied to the skin or mucosa once to 5 or 6 times per day in an effective amount for a target effect according to the age of the user (human), the gender, the intended use, the condition of the affected part of the skin, etc. The period of use of the composition of the present invention is not limited, but it is preferably used continuously to keep the effect of cell growth promotion and/or hyaluronic acid production promotion effectively, and the composition may be used, for example, for one month (preferably 2 months or more).

By promoting cell growth and / or promoting hyaluronic acid production, the composition of the present invention may exhibit the effects of anti-aging, moisturizing, anti-wrinkling, anti-sagging, hair growth, prevention of hair loss, wound healing, prevention/treatment of atopic dermatitis, prevention/treatment of senile xerosis and the like. Thus, the composition of the present invention may be used as a cosmetic or an externally-applied medical/quasi-medical drug for the purpose of anti-aging, moisturizing, anti-wrinkling, anti-sagging, hair growth, prevention of hair loss, wound healing, prevention/treatment of atopic dermatitis, prevention/treatment of senile xerosis and the like.

One aspect of the present invention includes a composition which does not contain acetylglucosamine, glucuronic acid, a salt of glucuronic acid, or a derivative thereof which are proposed to be blended into a cosmetic and the like in combination with a phosphate ester of uridine by JP 2014-88329 A.

As used herein, the derivative of acetylglucosamine has the following chemical formula (1): 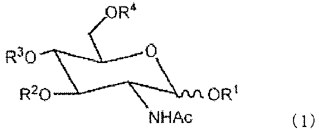 (R¹ is a hydrogen atom or an alkyl group having 2 to 18 carbon atoms. R², R³, and R⁴ are a hydrogen atom or an acyl group having 2 to 18 carbon atoms, and all may be the same or any may be different. The configuration at position 1 may be either α or β. Provided that all of R¹, R², R³, and R⁴ must not be hydrogen atoms.)

Specifically, the derivative are octyl(2-acetamide-2-deoxy)β-D-glucopyranoside, 2-acetamide-1,3,4,6-tetra-*O-*acetyl-2-deoxy-β-D-glucopyranoside, 2-acetamide-2-deoxy-6-*O*-octanoyl-α-D-glucopyranose, octyl(2-acetamide-2-deoxy-6-*O*-octanoyl)β-D-glucopyranoside, butyl(2-acetamide-2-deoxy)β-D-glucopyranoside, pentyl(2-acetamide-2-deoxy)β-D-glucopyranoside, lauryl(2-acetamide-2-deoxy)β-D-glucopyranoside, 2-acetamide-2-deoxy-6-*O*-palmitoyl-α-D-glucopyranose, geranyl(2-acetamide-2-deoxy)β-D-glucopyranoside, ethyl(2-acetamide-3,4,6-tri-*O*-acetyl-2-deoxy)β-D-glucopyranoside, pentyl(2-acetamide-3,4,6-tri-*O-*acetyl-2-deoxy)P-D-glucopyranoside, octyl(2-acetamide-3,4,6-tri-*O*-acetyl-2-deoxy)β-D-glucopyranoside, and geranyl(2-acetamide-3,4,6-tri-*O*-acetyl-2-deoxy)β-D-glucopyranoside.

The salts of glucuronic acid are potassium salt, sodium salt, and other physiologically acceptable salts. The derivatives of glucuronic acid are glucuronolactone, and glucuronoxylan.

Another aspect of the present invention includes a composition which does not contain a purine nucleic acid-related substance which are proposed to be blended into a cosmetic and the like in combination with a phosphate ester of uridine by WO 2003/084485 and WO 2005/034902.

In the present specification, purine nucleic acid-related substances are adenine, adenosine, adenosine phosphate [adenosine 2'-monophosphate, adenosine 3'-monophosphate, adenosine 5'-monophosphate, adenosine 5'-diphosphate, adenosine 5'-triphosphate, cyclic adenosine phosphate, adenylosuccinic acid, nicotinamide adenine monodinucleotide (NMN), nicotinamide adenine dinucleotide (NAD), nicotinamide adenine dinucleotide phosphate (NADP), flavin adenine dinucleotide (FAD)], metabolites thereof (hypoxanthine, inosine, inosinic acid), and salts thereof; guanine, guanosine, guanosine phosphate [guanosine 3'-monophosphate, guanosine 5'-monophosphate, guanosine 5'-diphosphate, and guanosine 5'-triphosphate, and the like], metabolites thereof [xanthylic acid, xanthin], and salts thereof. The above salts are alkali metal salts such as sodium salts, potassium salts; alkaline earth metal salts such as calcium salts, magnesium salts, barium salts; basic amino acid salts such as arginine, lysine; ammonium salts such as ammonium salts, tricyclohexylammonium salts; various kinds of alkanolamine salts such as monoethanolamine salts, diethanolamine salts, triethanolamine salts, monoisopropanolamine salts, diisopropanolamine salts, and triisopropanolamine salts.

The present invention provides a method for increasing skin moisture retention, a method for reducing wrinkle, a method for reducing skin-sag, a method for treating a wound, a method for preventing hair loss, a method for promoting hair growth, a method for preventing/treating atopic dermatitis, a method for promoting cell growth (for example hyaluronic acid-producing cell (for example keratinocyte, fibroblast), dermal papilla cell), and a method for promoting hyaluronic acid production. The method is carried out by applying a phosphate ester of uridine or a physiologically acceptable salt thereof to the skin.

According to the method of the invention, the application of the substance to the skin can be achieved by spreading, spraying, or sticking of the composition of the present invention to the skin.

In the method of the invention, there is no limitation on the frequencies with which a phosphate ester of uridine or a physiologically acceptable salt thereof is applied to the skin and also the application amounts thereof are not limited. For example, a phosphate ester of uridine or a physiologically acceptable salt thereof is applied to the skin in an appropriate amount from one to five or six times per day according to the age of the application target, the gender, the intended use, the condition of the affected part of the skin, etc. For example, when the method of the invention is carried out by using the composition of the present invention, a single dose can be suitably adjusted such that the amount of the composition applied to the skin is within the range of 0.5 to 10 mg/cm². The period of use of the method of the present invention is not limited, but it is preferably to be used continuously for a effectively-sustaining promotion of cell growth and a effectively-sustaining promotion of hyaluronic acid production, for example, for one month (preferably for two months or more).

### Example

The present invention is explained in further detail with reference to Examples and Test Examples. However, the scope of the invention is not limited to these Examples.

In the following Test Examples, ''w/v%'' means a weight (g) contained in 100 mL.

### [Example 1] Lotion (pH6.5)

| | |
|---|---|
| UMP2Na | 3.0(% by weight) |
| Polyoxyethylene(E.O.60) hydrogenated castor oil | 0.7 |
| Ethanol | 5.0 |
| Glycerin | 2.0 |
| Antiseptic | Suitable quantity |
| Flavor | Suitable quantity |
| pH adjuster | Suitable quantity |
| Purified water | Balance |
| Total | 100.0 % by weight |

A lotion is prepared according to the above formulation in a routine manner.

### [Example 2] Emulsion (pH6.5)

| | |
|---|---|
| UMP2Na | 1.5(% by weight) |
| Carboxyvinyl polymer | 0.3 |
| Decaglyceryl monomyristate | 2.0 |
| Squalane | 5.0 |
| Ethanol | 1.0 |
| Glycerin | 6.0 |
| Antiseptic | Suitable quantity |
| pH adjuster | Suitable quantity |
| Purified water | Balance |
| Total | 100.0 % by weight |

### [Example 3] Hair restorer (pH7.0)

| | |
|---|---|
| UMP2Na | 3(% by weight) |
| Polyoxyethylene polyoxypropylene decyl tetradecyl ether | 0.5 |
| Ethanol | 30 |
| 1,3-Butylene glycol | 2.5 |
| Antiseptic | Suitable quantity |
| Flavor | Suitable quantity |
| pH Adjuster | Suitable quantity |
| Purified water | Balance |
| Total | 100.0 % by weight |

A hair restorer is prepared according to the above formulation in a routine manner.

### <Test Example 1: Evaluation of hyaluronic acid production promoting effect of UMP2Na and uridine on cultured human epidermal keratinocytes and dermal fibroblasts>

Pre-cultured primary-cultured human epidermal keratinocytes (manufactured by Kurabo Industries, Ltd.) were cultured in a growth factor-containing EpiLife liquid medium (manufactured by ThermoFisher Scientific) in a 96-well microplate, and then the medium was replaced with the growth-factor-free medium containing an each concentration (0, 10⁻⁶, 10⁻⁵, 10⁻⁴, 10⁻³, 10⁻² mol/L) of UMP2Na or uridine.

Pre-cultured primary-cultured dermal fibroblasts (manufactured by Kurabo Industries, Ltd.) were cultured in a serum-containing Medium106 liquid medium (manufactured by ThermoFisher Scientific) in a 96-well microplate, and then the medium was replaced with the serum-free medium containing an each concentration (0, 10⁻⁶, 10⁻⁵, 10⁻⁴, 10⁻³, 10⁻² mol/L) of UMP2Na or uridine.

These were cultured for 48 and 72 hours under a condition of 37 °C and 5% CO₂. After the culturing, the culture supernatant in each well was collected, and the amount of hyaluronic acid in the culture supernatant was detected using a hyaluronic acid quantification kit (manufactured by Cosmo Bio) and measured with a microplate reader.

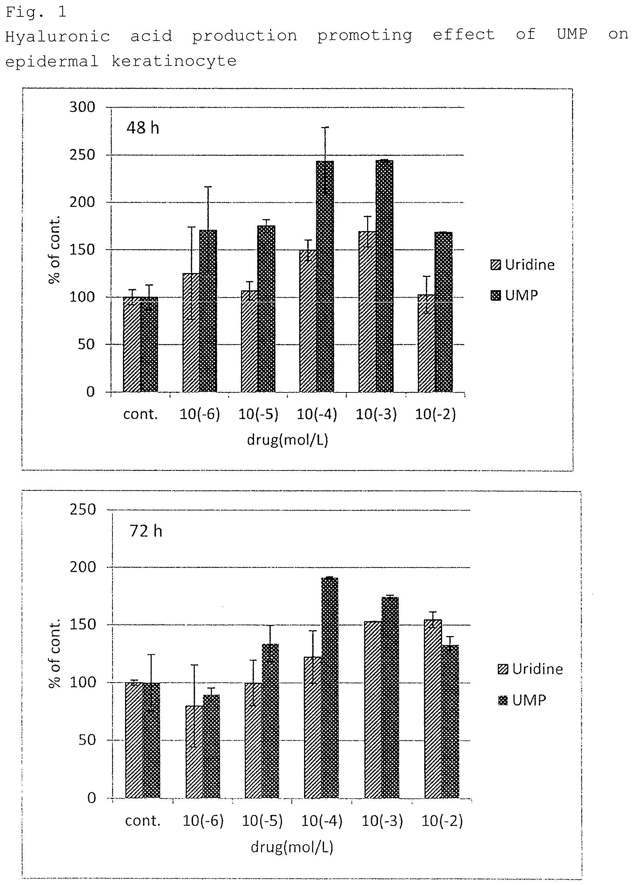

FIG. 1 shows the results of epidermal keratinocytes.

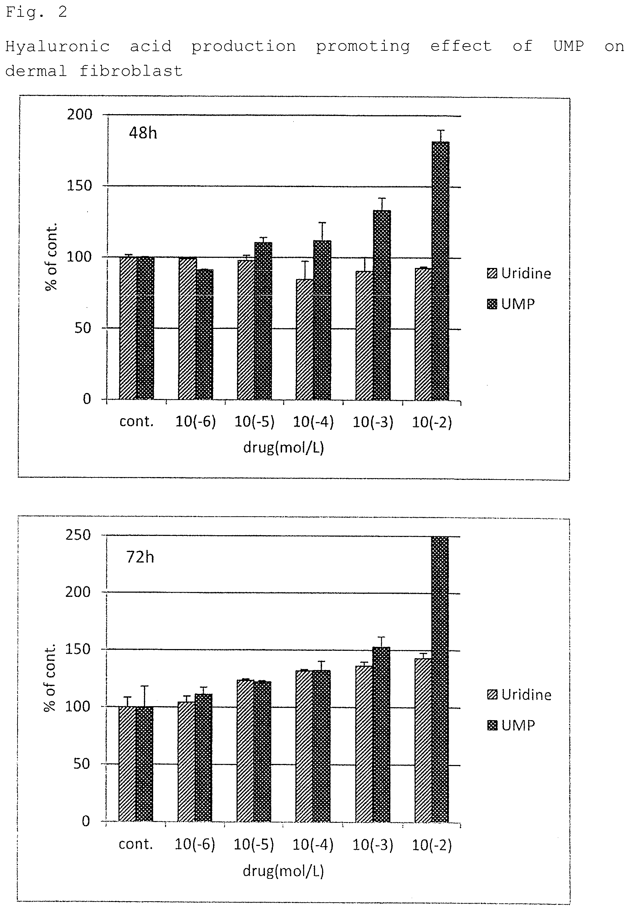

Fig. 2 shows the results of dermal fibroblasts.

### <Test Example 2: Evaluation of promoting effect of UMP2Na and uridine on cultured human dermal fibroblasts growth>

Pre-cultured primary-cultured human dermal fibroblasts (manufactured by Kurabo Industries, Ltd.) were cultured in a serum-containing Medium106 liquid medium (manufactured by ThermoFisher Scientific) in a 96-well microplate. After confirming the stable adhesion, the medium was replaced with the same medium containing an each concentration (0, 10⁻⁶, 10⁻⁵, 10⁻⁴, 10⁻³, 10⁻² mol/L) of UMP2Na or uridine. This was cultured for 48, 72, and 96 hours under a condition of 37 °C and 5% CO₂. After each culturing time, the number of cells in each well was detected using WST-1 reagent (manufactured by Takara Bio Inc.) and then measured with a microplate reader.

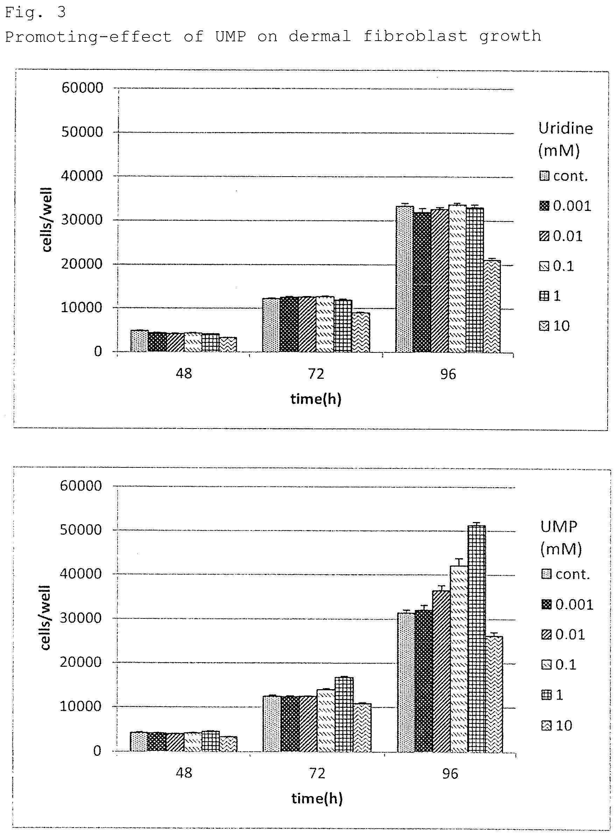

The results are shown in Figure 3.

### <Test Example 3: Evaluation of promoting effect of UMP2Na on cultured human dermal papilla cell growth>

Pre-cultured primary-cultured human dermal papilla cells (manufactured by Takara Bio Inc.) were cultured in a dermal papilla cell growth medium (manufactured by Takara Bio Inc.) in a 96-well microplate. After confirming the stable adhesion, the medium was replaced with the medium containing various concentrations of the drug.

This was cultured for 72, 96, and 120 hours under a condition of 37 °C and 5% CO₂. After each culturing time, cell nuclei were stained, and the number of cells in each well was counted.

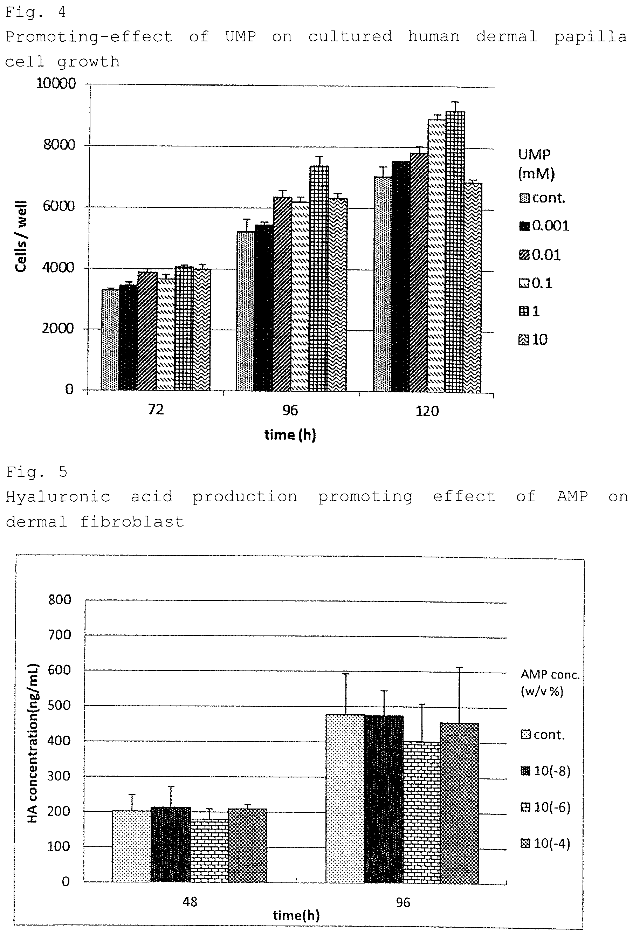

The results are shown in Figure 4.

### <Test Example 4: Evaluation of hyaluronic acid production promoting effect of adenosine monophosphate disodium on cultured human dermal fibroblasts>

Pre-cultured primary-cultured dermal fibroblasts (manufactured by Kurabo Industries, Ltd.) were cultured in a serum-containing Medium106 liquid medium (manufactured by ThermoFisher Scientific) in a 96-well microplate. After the culturing, the medium was replaced with the serum-free medium containing an each concentration (0, 10⁻⁸, 10⁻⁶, 10⁻⁴ w/v%) of adenosine monophosphate disodium (AMP2Na). This was cultured for 48 and 96 hours under a condition of 37 °C and 5% CO₂. After the culturing, the culture supernatant in each well was collected, and the amount of hyaluronic acid in the culture supernatant was detected using a hyaluronic acid quantification kit (manufactured by Cosmo Bio) and measured with a microplate reader.

The results are shown in Figure 5.

## Claims

1. A composition for external use, comprising a phosphate ester of uridine or a physiologically acceptable salt thereof as an active ingredient for moisturizing, anti-wrinkling, anti-sagging, wound healing, prevention of hair loss, hair growth, or prevention/treatment of atopic dermatitis.

2. The composition according to Claim 1, comprising a phosphate ester of uridine or a physiologically acceptable salt thereof as an independent active ingredient.

3. The composition according to Claim 1 or 2, comprising no effective amount of any other active ingredient for moisturizing, anti-wrinkling, anti-sagging, wound healing, prevention of hair loss, hair growth, or prevention/treatment of atopic dermatitis.

4. The composition according to any one of Claims 1 to 3, wherein a phosphate ester of uridine or a physiologically acceptable salt thereof is selected from uridine monophosphate, uridine diphosphate, uridine triphosphate, and a physiologically acceptable salt thereof.

5. The composition according to any one of Claims 1 to 4, wherein a phosphate ester of uridine or a physiologically acceptable salt thereof is selected from uridine monophosphate and a physiologically acceptable salt thereof.

6. The composition according to any one of Claims 1 to 5, wherein a phosphate ester of uridine or a physiologically acceptable salt thereof is selected from uridine 5'-monophosphate and a physiologically acceptable salt thereof.

7. The composition according to any one of Claims 1 to 6, wherein the composition is for use on skin.

8. The composition according to any one of Claims 1 to 7, wherein the composition is used for promoting cell growth or promoting hyaluronic acid production.

9. The composition according to any one of Claims 1 to 8, which is used for a cosmetic, a medical drug for external use, or a quasi-medical drug for external use.

10. A composition for external use, comprising a phosphate ester of uridine or a physiologically acceptable salt thereof as an active ingredient for promoting cell growth or promoting hyaluronic acid production.

11. The composition according to Claim 10, comprising a phosphate ester of uridine or a physiologically acceptable salt thereof as an independent active ingredient.

12. The composition according to Claim 10 or 11 , comprising no effective amount of any other active ingredient for promoting cell growth or promoting hyaluronic acid production.

13. The composition according to any one of Claims 10 to 12, wherein a phosphate ester of uridine or a physiologically acceptable salt thereof is selected from uridine monophosphate, uridine diphosphate, uridine triphosphate, and a physiologically acceptable salt thereof.

14. The composition according to any one of Claims 10 to 13, wherein a phosphate ester of uridine or a physiologically acceptable salt thereof is selected from uridine monophosphate and a physiologically acceptable salt thereof.

15. The composition according to any one of Claims 10 to 14, wherein a phosphate ester of uridine or a physiologically acceptable salt thereof is selected from uridine 5' -monophosphate and a physiologically acceptable salt thereof.

16. The composition according to any one of Claims 10 to 15, wherein the composition is for use on skin.

17. The composition according to any one of Claims 10 to 16, which is used for a use selected from moisturizing, anti-wrinkling, anti-sagging, wound healing, prevention of hair loss, hair growth, and prevention/treatment of atopic dermatitis.

18. The composition according to Claim 17, comprising no effective amount of any other active ingredient for moisturizing, anti-wrinkling, anti-sagging, wound healing, prevention of hair loss, hair growth, or prevention/treatment of atopic dermatitis.

19. The composition according to any one of Claims 10 to 18, which is used for a cosmetic, a drug for external use, or a quasi-drug for external use.
